Europäisches Patentamt

European Patent Office

Office européen des brevets

(11) Publication number: **0 233 704 B1**

## EUROPEAN PATENT SPECIFICATION

(45) Date of publication of patent specification: **15.07.92**  (51) Int. Cl.⁵: **A61F 5/44**, A61F 13/15

(21) Application number: **87300450.1**

(22) Date of filing: **20.01.87**

(54) Disposable diaper having wide tapered fastening tapes.

(30) Priority: **21.01.86 US 821100**

(43) Date of publication of application:
**26.08.87 Bulletin 87/35**

(45) Publication of the grant of the patent:
**15.07.92 Bulletin 92/29**

(84) Designated Contracting States:
**AT BE CH DE ES FR GR IT LI LU NL SE**

(56) References cited:
EP-A- 0 158 490    GB-A- 2 080 093
US-A- 2 833 282    US-A- 3 848 594
US-A- 3 860 003    US-A- 4 209 016

(73) Proprietor: **THE PROCTER & GAMBLE COM-
PANY
One Procter & Gamble Plaza
Cincinnati Ohio 45202(US)**

(72) Inventor: **Burkhard, Harold Richard
8070 Peacock Drive
Cincinnati Ohio 45239(US)**
Inventor: **Buell, Kenneth Barclay
218 Brocdorf Drive
Cincinnati Ohio 45215(US)**

(74) Representative: **Gibson, Tony Nicholas et al
Procter & Gamble (NTC) Limited Whitley
Road
Longbenton Newcastle upon Tyne NE12
9TS(GB)**

## Description

### TECHNICAL FIELD

This invention relates to disposable diapers having elasticized leg openings, and more particularly to an improved side closure for such disposable diapers. Still more particularly, this invention relates to an improved fastening tape having a manufacturer's end (i.e., the end affixed to the diaper by the manufacturer) and a user's end (i.e., the end that the user affixes to the diaper when fitting it on the wearer) where the user's end is relatively wide at the longitudinal sides of the diaper and is tapered to a more narrow width at its distal end. As used herein, the term "fastening tape" is intended to describe any tape having an adhesive or mechanical closure system known in the art.

### BACKGROUND OF THE INVENTION

Disposable diapers are well known articles of manufacture that are worn by infants and incontinent persons. Disposable diapers are worn about the lower torso and are intended to absorb and contain urine and feces, thereby preventing the urine and feces from soiling, wetting, or otherwise contaminating articles (e.g., clothing, bedding, etc.) that come into contact with the diaper wearer.

In general, disposable diapers have the same basic structure comprising an absorbent core encased between a liquid permeable, user-contacting topsheet and a liquid impermeable backsheet. The prior art, of course, teaches numerous variations of and elements in addition to the basic topsheet, backsheet, and absorbent core arrangement. For example, an improvement in the performance of disposable diapers has been achieved by the addition of elastic means along portions of the diaper contacting the wearer's thigh, thereby providing elasticized leg openings when the diaper is worn.

When using a disposable diaper having elasticized leg openings, the diaper user fits the diaper on the wearer and fastens it about the wearer's waist thereby effecting a side closure. Fitting the diaper about the wearer usually requires the front and back waist portions of the diaper to overlap each other. As the diaper is worn, the movements of the wearer and the forces induced by the elastic means at the leg openings tend to cause the overlapping front and back waist portions to shift position relative to each other. In other words, the overlapping front and back waist portions are subjected to forces that tend to cause them to assume a position relative to each other that is different from the position they assume when the diaper is initially fitted to the wearer. Unless such shifting is limited, the fit of the diaper about the legs and waist and its containment characteristics are degraded as the diaper is worn.

A number of concepts have been proposed for fastening a disposable diaper about the waist of the wearer. For example, U.S. Patent 3,848,594, issued to K. B. Buell on November 19, 1974, teaches a tape fastening system for effecting a side closure having an improved manufacturer's joint, which is hereinafter referred to as a Y-bond.

U.S. Patent 3,967,622, Cepuritis, issued July 6, 1976, and U.S. Patent 4,010,754, Pieniak, issued March 8, 1977, disclose disposable diapers having fastening tapes with wide or flared manufacturer's ends to distribute stresses and minimize tearing of the backsheet. The user's ends of these fastening tapes appear to be of standard width and are not tapered.

None of the foregoing patents are specifically directed to disposable diapers having elasticized leg openings and the special problems of maintaining good fit characteristics associated with such diapers. U.S. Patent 4,253,461, issued to D. L. Strickland et al. on March 3, 1981, is directed to a disposable diaper having elasticized leg openings and achieves an improved side closure by providing separate fastening means at the waist and at the thigh. A disposable diaper according to the preamble of Claim 1 is known from the document US-A-3 860 003.

The disposable diapers of the prior art lack the aspects of the present invention wherein the fastening tapes have a user's end that is wide at the longitudinal sides of the diaper to minimize relative motion between the front and back waist portions and gapping about the legs, and that is tapered toward its distal end to increase wearing comfort and to make the tapes easier to handle and apply, i.e. like conventional fastening tapes.

It is therefore an object of the present invention to provide improved fastening tapes for a disposable diaper having elasticized leg openings.

A further object of the present invention is to provide fastening tapes that better maintain the fit of the diaper at the waist and at the elasticized leg openings.

An additional object of the invention is to provide a disposable diaper having elasticized leg openings which has improved urine and feces containment characteristics.

Another object of the invention is to provide the above improved fit and containment characteristics while maintaining the wearing comfort and ease of handling characteristics of conventional fastening tapes.

These and other objects of the invention will be more readily apparent when considered in connection with the following description and the accompanying drawings.

## SUMMARY OF THE INVENTION

According to the present invention, a disposable diaper having elasticized leg openings is manufactured such that an absorbent means is encased between a liquid permeable topsheet and a liquid impermeable backsheet. A portion of the contractive force generated by the elastic means used at the leg openings is directed into the waist portions of the diaper. These forces, together with movements of the diaper wearer, tend to cause shifting of the front and back waist portions. Shifting, as used herein, is movement of the front and back waist portions relative to each other from the position they assume when the diaper is initially applied. When such relative motion occurs, the fit about the wearer's legs and waist degenerates making the diaper less effective in containing waste products.

The disposable diaper of the present invention is provided with fastening tapes having a user's end that is wide at the longitudinal sides of the diaper and that is tapered toward its distal end. When the diaper is placed around the waist of the wearer, the waist portions of the diaper are made to overlap and are then affixed to each other by the fastening tapes. The wide user's end of the fastening tapes tensions and secures more of the overlapping waist portions extending from the waistband edge to the elasticized leg openings. This minimizes shifting of the overlapping waist portions, and any resulting waistband rollover or gapping at the legs and waist, so that a good fit about the legs and waist is maintained.

Tapering of the user's end of the fastening tapes reduces tape stiffness, bulkiness and contact pressure, which increases wearing comfort and minimizes any redmarking or other irritation of the skin. Tapering of the user's end of the tapes also makes them easier to handle and apply, i.e. to conveniently tension, position and fasten or refasten the tapes without inadvertently "snagging" a sticky edge or folding a tape onto itself. Finally, depending upon the degree of tapering of the user's end and where the tapering begins, a relatively wide fastening or adhesion zone can still be obtained adjacent the longitudinal sides of the diaper, which is preferred from the standpoint of minimizing any shifting of the overlapped waist portions.

The resulting disposable diaper thereby exhibits improved fit and containment characteristics while maintaining the wearing comfort and ease of handling characteristics of conventional fastening tapes.

## BRIEF DESCRIPTION OF THE DRAWINGS

Figure 1 is a partially cutaway perspective view of an elasticized disposable diaper of the present invention having wide tapered fastening tapes.

Figure 2 is a perspective view showing the disposable diaper of Figure 1 in the configuration it would assume when placed on a wearer.

Figure 3 is an enlarged fragmentary plan view of an alternate fastening tape of the present invention.

Figure 4 is an enlarged plan view showing a preferred repeating pattern for making fastening tapes of the present invention.

Figure 5 is an enlarged sectional view taken along line 5-5 of Figure 4.

Figures 6 and 7 are enlarged plan views showing repeating patterns for making alternate fastening tapes of the present invention.

## DETAILED DESCRIPTION OF THE INVENTION

Referring now to the drawings, there is shown a preferred embodiment of the present invention as it would be used in a disposable diaper intended to be worn by an infant. As used herein, the term "disposable diaper" refers to a garment generally worn by infants or incontinent persons, which is drawn up between the legs and fastened about the waist of the wearer and further, which is intended to be discarded after a single use (i.e. it is not intended to be laundered or otherwise restored and reused).

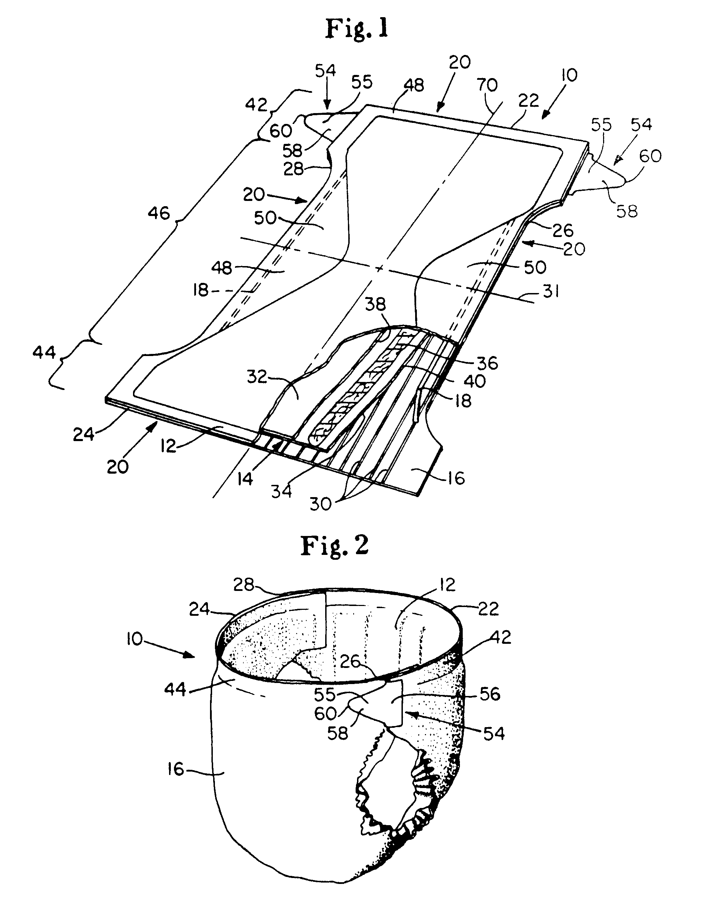

Figure 1 is a partially cut away perspective view of the disposable diaper 10 of the present invention prior to its being folded and placed on the diaper wearer by the diaper user. As can be seen in Figure 1, a preferred diaper 10 basically comprises a liquid permeable topsheet 12, an absorbent means 14, a liquid impermeable backsheet 16 and elastic members 18. While the topsheet 12, absorbent means 14, liquid impermeable backsheet 16, and elastic members 18 may be assembled in a variety of well known configurations, a preferred disposable diaper configuration is described generally in U.S. Patent 3,860,003.

Figures 1 and 2 show a preferred embodiment of the diaper 10 in which the topsheet 12 and the backsheet 16 are coextensive and have length and width dimensions generally larger than those of the absorbent means 14. The topsheet 12 is superposed on the backsheet 16 thereby forming a periphery 20 of diaper 10. The periphery 20 defines the outer periphery or, in other words, the outer extent of the diaper 10. The periphery 20 comprises first end 22, second end 24, first longitudinal side 26, and second longitudinal side 28.

The topsheet 12 may be affixed to the backsheet 16 in any suitable manner as is well known in the diaper manufacturing art. In a preferred embodiment, a multiplicity of longitudinal adhesive bands 30 of hot-melt adhesive are applied along the full length of the backsheet 16 generally parallel to the longitudinal centerline 70 of the backsheet 16. The longitudinal adhesive bands 30 serve to affix the topsheet 12 to the backsheet 16 at those points where these three components come together. The extent and location of the points where the topsheet 12, backsheet 16, and longitudinal adhesive bands 30 come together will depend on the spacing between the longitudinal adhesive bands 30 and on the distance the topsheet 12 and the backsheet 16 extend beyond the absorbent means 14. The number of longitudinal adhesive bands 30 and the spacing therebetween should be sufficient to securely bond the topsheet 12 to the backsheet 16 in the area between the periphery 20 and the edge of the absorbent means 14.

A hot-melt adhesive suitable for use as longitudinal adhesive bands 30 is manufactured by Eastman Chemical Products Company, of Kingsport, Tennessee and marketed under the tradename Eastobond A-3. It will be noted that the above described manner of affixing the topsheet 12 to the backsheet 16 causes the topsheet 12 to be affixed to the backsheet 16 intermittently along the first and second ends, 22 and 24. The absorbent means 14 is thereby encased between the topsheet 12 and the backsheet 16. Of course, many alternative methods of affixing the topsheet 12 to the backsheet 16 may be used with satisfactory results. For example, the topsheet 12 may be affixed to the backsheet 16 indirectly rather than directly as shown in Figure 1. Thus, an intermediate member may be used to affix the topsheet 12 to the backsheet 16.

The diaper 10 has first and second waist portions 42 and 44 extending, respectively, from the first end 22 and the second end 24 of the diaper periphery 20 toward the lateral centerline 31 of the diaper 10, a distance from about 1/5 to about 1/3 the length of the diaper. The waist portions 42 and 44 comprise those portions of the diaper 10 which, when worn, encircle the waist of the wearer. The crotch portion 46 is that portion of the diaper 10 between first and second waist portions 42 and 44, and comprises that portion of the diaper 10 which, when worn, is positioned between the legs of the wearer and covers the lower torso of the wearer.

The absorbent means 14 may be any means which is generally compressible, conformable, non-irritating to the wearer's skin, and which is capable of absorbing and retaining liquids. A preferred absorbent means 14 has first and second opposed faces 32 and 34, respectively, and comprises an absorbent layer 36 and first and second tissue layers 38 and 40, respectively. The first and second tissue layers 38 and 40 overlay the major surfaces of the absorbent layer 36 to form the first and second opposed faces 32 and 34 of the absorbent means 14.

The absorbent layer 36 is intended to absorb and contain liquid and may be manufactured in a wide variety of sizes and shapes (e.g., rectangular, hourglass, etc.) and from a wide variety of liquid absorbent materials commonly used in disposable diapers, such as comminuted wood pulp which is generally referred to as airfelt. Other liquid absorbing materials may also be used in the manufacture of the absorbent layer 36 such as a multiplicity of plies of creped cellulose wadding, polymeric gelling agents, absorbent foams or sponges, or any equivalent material or combination of materials. The total absorbent capacity of the absorbent layer 36 should, however, be compatible with the design liquid loading in the intended use of the disposable diaper 10. Further, the size and absorbent capacity of the absorbent layer 36 may be varied to accommodate wearers ranging from infants through adults.

The preferred embodiment of diaper 10 illustrated in Figures 1 and 2 has an hourglass shaped absorbent layer 36, and is intended to be worn by infants ranging in weight from 12 to 26 pounds (5 kg to 12 kg) The absorbent layer 36 is, therefore, a batt of airfelt approximately 16 inches (41 cm) long when measured along the longitudinal centerline, approximately 12 inches (32 cm) across the first and second ends 22 and 24, and approximately 4 inches (10 cm) across the narrowest part of the crotch portion 46. The absorptive capacity of the airfelt used for the absorbent layer 36 is sufficient to absorb and retain from 8 to 16 grams of water per gram of absorbent material. Accordingly, the airfelt used in the preferred embodiment shown in Figures 1 and 2 weighs from 30 to 56 grams and has a generally uniform caliper. It should be understood, however, that the size, shape, configuration, and total absorbent capacity of the absorbent layer 36 may be varied to accommodate wearers ranging from infants through adults. Therefore, the dimensions, shape, and configuration of the absorbent layer 36 may be varied (e.g. the absorbent layer 36 may have a varying caliper, or a hydrophilic gradient, or may contain polymeric gelling agents).

The first and second tissue layers, 38 and 40, are intended to improve the tensile strength of the absorbent core 14 and to reduce the tendency of the absorbent layer 36 to split, lump or ball when wetted. The first and second tissue layers, 38 and 40, also help to improve lateral wicking of liquids, thereby providing a more even distribution of liquid in the absorbent layer 36. While a number of materials and manufacturing techniques may be used to manufacture the first and second tissue layers, 38 and 40, satisfactory results have been obtained with sheets of wet strength tissue paper having a basis weight of approximately 12 pounds per 3000 square feet (16 grams per square meter) and having an air permeability of approximately 100 cubic feet per minute per square foot (30 cubic meters per minute per square meter) over a 0.5 inches (13 mm) water pressure drop. While the first and second tissue layers, 38 and 40, are preferably cotermincus with the absorbent layer 36, they may have different dimensions, a different configuration, or they may be omitted entirely.

The absorbent means 14 is superposed on the backsheet 16 and is preferably affixed thereto by any means known in the diaper art. For example, the absorbent core 14 can be secured to the backsheet 16 by a uniform continuous layer of adhesive, a patterned layer of adhesive, or an array of lines or spots of adhesive. In the preferred embodiment illustrated in Figures 1 and 2, the longitudinal adhesive bands 30 are used to affix the absorbent core 14 to the backsheet 16.

The backsheet 16 is impermeable to liquids and prevents liquids absorbed by the absorbent means 14 from wetting the undergarments, clothing, bedding, and other objects which contact the wearer of the disposable diaper 10. Preferably the backsheet 16 is a polyethylene film of from 0.0005 to 0.002 inches (0.012 to 0.051 mm) thick, although other flexible, liquid impermeable materials may also be used. As used herein, the term "flexible" refers to materials which are compliant and which readily conform to the shape and contours of the human body. A suitable polyethylene film is manufactured by Monsanto Chemical Company and marketed in the trade as Film No. 8020. The backsheet 16 is preferably embossed and/or matte finished to provide a more clothlike appearance. Further, the backsheet 16 may have passages which permit vapors to escape from the absorbent means 14 while still preventing liquid from passing through the backsheet 16.

In a preferred embodiment, the backsheet 16 has a modified hourglass shape extending beyond the absorbent layer 36 a minimum distance of at least 0.5 inches (1.3 cm) around the entire diaper periphery 20. The marginal portion 48 is that portion of the diaper 10 between the diaper periphery 20 and the edge of the absorbent layer 36 and comprises longitudinal marginal portions 50 adjacent first and second longitudinal sides 26 and 28, respectively, in the crotch portion 46.

The topsheet 12 is compliant, soft feeling, and non-irritating to the wearer's skin and prevents the wearer of diaper 10 from contacting the absorbent core 14. Further, the topsheet 12 is liquid permeable permitting liquids to readily penetrate through its thickness. A suitable topsheet 12 may be manufactured from a wide range of materials, such as natural fibers (e.g., wood or cotton fibers), synthetic fibers (e.g., polyester, polypropylene), or a combination thereof. Alternatively, the topsheet 12 may be a foam, such as the reticulated foams which are well known in the art or any of the formed films which are also well known in the art.

A number of manufacturing techniques can be used to manufacture the topsheet 12. For example, the topsheet 12 may be woven, nonwoven, spunbonded, carded, or the like. A preferred topsheet 12 is carded, and thermally bonded by means well known to those skilled in the nonwoven fabrics art. Preferably the topsheet 12 has a weight of from 21.4 to 29.75 grams per square meter, a minimum dry tensile strength of at least 400 grams per centimeter in the machine direction and a wet tensile strength of at least 55 grams per centimeter in the cross machine direction.

The elastic members 18 are affixed to the diaper 10 along both longitudinal marginal portions 50 so that they tend to draw and hold the diaper 10 against the legs of the wearer. Thus, when worn the diaper 10 will have elasticized leg openings. While this result may be accomplished by any of several means as are well known in the diaper art, a particularly preferred diaper construction incorporating elastic strips is described in detail in the hereinbefore referenced U.S. Patent 3,860,003. In addition, a method and apparatus suitable for manufacturing a disposable diaper having elastic leg bands are described In U.S. Patent 4,081,301.

Relating the teachings of U.S. Patent 3,860,003 to the preferred embodiment shown in Figures 1 and 2, it can be seen that elastic members 18 are operatively associated with both longitudinal marginal portions 50 in the crotch portion 46 in an elastically contractible condition so that in a normally unrestrained configuration the elastic members 18 effectively contract or gather the longitudinal marginal portions 50. (As used herein, the term "operatively associated with" refers to two or more components which act together.)

The elastic members 18 can be operatively associated with the longitudinal marginal portions 50 in an elastically contractible condition in at least two ways. For example, the elastic members 18 can be stretched and while in the stretched condition affixed to the uncontracted and unstretched longitudinal marginal portions 50. Alternatively, the longitudinal marginal portions 50 can be contracted (e.g., by pleating) and then the unstretched elastic members 18 affixed to the contracted longitudinal marginal portions 50.

The elastic members 18 can be affixed to the longitudinal marginal portions 50 in any of several ways which are well known in the art. For example, the elastic members 18 can be ultrasonically bonded or heat sealed into the longitudinal marginal portions 50 using a variety of bonding patterns or the elastic members 18 can simply be glued to the longitudinal marginal portions 50.

In the preferred embodiment illustrated in Figures 1 and 2, the elastic members 18 are affixed using an adhesive to a portion of the backsheet 16 in the longitudinal marginal portions 50. A suitable adhesive will be flexible and of sufficient adhesiveness to hold the elastic members 18 to the backsheet 16 while the elastic members 18 are stretched. An adhesive which has been used with satisfactory results is manufactured by Findley Adhesives Incorporated of Elm Grove, Wisconsin and marketed as Findley Adhesives 581-334-01.

Suitable elastic members 18 may be manufactured from a wide variety of elastic materials such as natural rubber, or elastomeric films such as Kraton, ethylene propylene monomer, and polyurethane.

In addition, the elastic members 18 may take a multitude of configurations. For example, the width of the elastic members 18 may be varied from 0.0015 inches to 1.0 inches (0.038 mm to 25 mm); the elastic members 18 may comprise a single strip of elastic material or may comprise several parallel or non parallel strips of elastic material; or the elastic members 18 may be rectilinear or curvilinear. The wide tapered tapes herein are particularly useful for preventing gapping at the elasticized leg openings caused by curvilinear elastic members.

One material which has been found to be suitable as an elastic member 18 is an elastic tape having a cross section of 0.007 inches by 0.25 inches (0.18 mm by 6.4 mm) and which is manufactured from natural rubber. Such a product is marketed by Easthampton Rubber Thread Company under the tradename L-1900 rubber compound. The preferred elastic member 18 produces a tensile force of 100 grams when stretched 100 percent from its relaxed condition.

The diaper 10 is provided with fastening tapes 54 for maintaining the first and second waist portions 42 and 44 in an overlapping configuration when the diaper 10 is worn. Thus, the diaper 10 is fitted to the wearer and a side closure formed.

More specifically, the fastening tapes 54 affix the first waist portion 42 to the second waist portion 44 thereby maintaining the first and second waist portions 42 and 44 in an overlapping configuration. Thus, the fastening tapes 54 must be affixed to both the first waist portion 42 and to the second waist portion 44 in a manner and with a strength that is sufficient to resist the forces acting to cause the first and second waist portions 42 and 44 to separate during wearing.

The fastening tapes 54 have a manufacturer's end 56 and a user's end 58. The manufacturer's end 56 is that end of fastening tapes 54 which the manufacturer of the diaper 10 affixes to the diaper 10 while the user's end 58 is that end of fastening tapes 54 which the user affixes to the diaper 10 when fitting it to the wearer. The manufacturer's end 56 can be affixed to the first waist portion 42 by any suitable methods known in the art, such as by using ultrasonic bonding, heat sealing, or pressure-sensitive adhesives, which are preferred. After fitting the diaper 10 about the waist of the wearer, the user's end 58 is affixed to the second waist portion 44 thereby causing the diaper 10 to encircle the waist of the wearer and effecting a side closure.

The user's end 58 of fastening tapes 54 should have a longitudinal width of from 1.5 inches to 4 inches (3.8 cm to 10.2 cm), preferably from 2 inches to 3 inches (5.1 cm to 7.6 cm), at the first and second longitudinal sides 26 and 28 of the diaper 10. In addition, the user's end 58 of fastening tapes 54 should be tapered such that its longitudinal width at the distal end 60 is less than 60%, preferably less than 50%, most preferably less than 40%, of its longitudinal width at the first and second longitudinal sides 26 and 28 of diaper 10. While the tapering of the user's end 58 can begin adjacent its distal end 60, it preferably begins adjacent the first and second longitudinal sides 26 and 28 of diaper 10 to provide larger reductions in tape stiffness, bulkiness and contact pressure. The user's end 58 preferably has a longitudinal width of from 0.5 inches to 1.5 inches (1.3 cm to 3.8 cm) at a distance of 0.375 inches (0.95 cm) from its distal end 60 to provide a convenient gripping zone for tensioning and fastening or refastening the tape 54. Finally, the tapering of the user's end 58 is preferably of a gradual nature, and preferably is of an equal degree on both sides of user's end 58 to provide lateral symmetry.

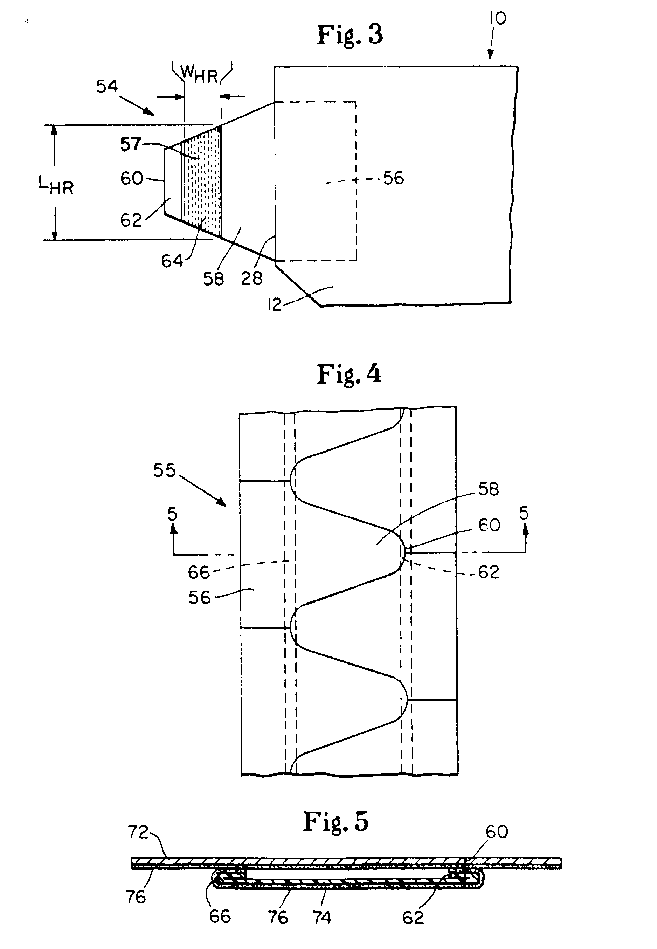

The user's end 58 of fastening tapes 54 preferably has a length in the lateral direction of from 0.5 inches (1.3 cm) to 2.5 inches (6.4 cm), more preferably from 1 inch (2.5 cm) to 2 inches (5.1 cm). In addition, the manufacturer's end 56 of fastening tapes 54 preferably has a longitudinal width about the same as the longitudinal width of the user's end at the longitudinal sides 26 and 28 of diaper 10. The manufacturer's end 56 preferably has a length in the lateral direction of from 0.5 inches (1.3 cm) to 2 inches (5.1 cm), more preferably from 0.75 inches (1.9 cm) to 1.5 inches (3.8 cm). Examples of such wide tapered tapes are shown in Figures 1, 2, 4, 6 and 7 in which the manufacturer's end 56 is rectangular in shape and the user's end 58 is sinusoidal-like in shape, and in Figure 3 in which the manufacturer's end 56 is rectangular in shape and the user's end 58 is trapezoidal in shape. It will be appreciated that other shapes (e.g., triangular or irregular shapes) can be selected for both the manufacturer's end and the user's end herein.

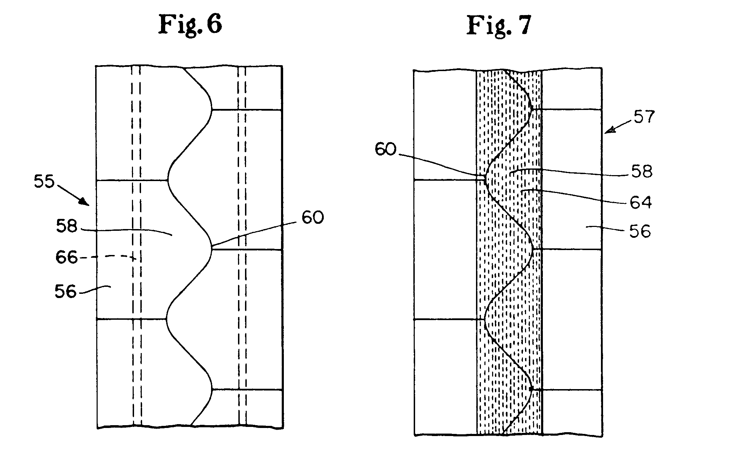

The fastening tapes 54 can comprise any of the well known means for achieving a side closure, such as pressure-sensitive adhesives or mechanical fasteners, (e.g., hooks, loops, buttons, or snaps). Preferred pressure-sensitive adhesive tapes 55 are illustrated in Figures 1, 2 and 4-6. Preferred mechanical fastening tapes 57 containing hooks are illustrated in Figures 3 and 7. (The corresponding loops that engage the hooks are not shown.)

The fastening tapes 54 can also comprise any of the well known configurations and constructions. For example, fastening tapes 54 can be single use tapes or multiple use tapes (i.e., refastenable). Preferred refastenable adhesive tapes containing a Y-bond are described in detail in U.S. Patent 3,848,594.

Fastening tapes 54 preferably have a relatively soft and flexible backing to increase wearing comfort and minimize any redmarking or other irritation of the skin in the hip region. Such tape backings preferably have a Taber stiffness in the longitudinal direction of less than 3 g•cm more preferably from 0.1 to 2.5, most preferably from 0.5 to 2 g•cm. As used herein, Taber stiffness is determined according to TAPPI standard T489 0S-76. The tape backings also preferably have a Taber stiffness within the above ranges in the lateral direction, and preferably have low tensile stretch in the lateral direction.

A particularly preferred tape backing is available from Minnesota Mining and Manufacturing Company, St. Paul, Minnesota, under the code number XPF 14.43.0. This backing has a Taber stiffness of 0.9 g•cm. When coated with a pressure-sensitive adhesive, it is available from Minnesota Mining and Manufacturing Company under the code number XPF 1.42.34.

Figure 3 is an enlarged fragmentary plan view of the wearer's side of the first waist portion 42 of an alternate diaper 10 of the present invention. In Figure 3, fastening tape 54 is a mechanical fastening tape 57 and comprises manufacturer's end 56, user's end 58, distal end 60, end tab 62, and hook region 64. Also shown is second longitudinal side 28 and topsheet 12 of diaper 10. Not shown is the loop region that engages hook region 64 (and the corresponding hook region 64 on the other mechanical fastening tape 57 of diaper 10) to maintain the first and second waist portions 42 and 44 in an overlapping configuration. While the loop region can assume varying sizes and shapes, it preferably comprises one or more patches placed across the outside of the second waist portion 44 to allow for maximum fit adjustment at the waist.

To minimize any possible contact between the hooks of the hook region 64 and the skin of the wearer, hook region 64 can be located, as in Figure 3, near the distal end 60 of the user's end 58 of mechanical fastening tape 57. However, it will be appreciated that shifting of the overlapping first and second waist portions 42 and 44 can best be prevented by locating the hook region 64 on the user's end 58 in the area immediately adjacent the second longitudinal side 28. In any event, the loop region should be positioned such that the distance from the first and second longitudinal sides 26 and 28 in the second waist portion 44 to the loop region is at least as large as the lateral width of hook region 64 ($W_{HR}$) to avoid contact between hook region 64 and the skin of the wearer. Hook region 64 can be as wide as user's end 58, but it preferably has a width of less than 0.75 inches (1.9 cm). Also, since the absorbent layer 36 of diaper 10 generally ends one inch (2.5 cm) from the longitudinal sides 26 and 28 in the second waist portion 44, the region therebetween (i.e. marginal portion 48) is a highly flexible area comprising topsheet 12 and backsheet 16 which can fold under during wearing of diaper 10. Thus, to prevent contact between hook region 64 and the skin if this flexible area is folded under, it is preferred that the loop region be inboard from the longitudinal side edges of absorbent layer 36 by a distance at least equal to the lateral width of hook region 64 ($W_{HR}$). Finally, the longitudinal width of the loop region is preferably no more than one inch (2.5 cm) larger than the longitudinal length of hook region 64 ($L_{HR}$) so that fastening automatically results in proper fitting of diaper 10.

Hook region 64 is preferably a patch of the material sold by Minnesota Mining and Manufacturing Company under the trade name Scotchmate SJ3492. The loop region is preferably a patch of the material Scotchmate SJ3491. The hook region 64 and loop region patches are preferably attached to the user's end 58 of mechanical fastening tape 57 and to the outside of the second waist portion 44, respectively, using a pressure-sensitive adhesive.

Figure 4 is an enlarged plan view showing a preferred repeating pattern for making fastening tapes 54 having a sinusoidal shaped user's end 58. This pattern results in little or no waste of tape material and simultaneously produces and orients both fastening tapes 54 for each diaper 10. Shown are particularly preferred refastenable adhesive tapes 55 comprising manufacturer's end 56, user's end 58, distal end 60, end tab 62, and Y-bond 66, which is described in the above incorporated U.S. Patent 3,848,594.

Figure 5 is an enlarged sectional view taken along lines 5-5 of Figure 4. It shows tape backing 72 and release tape 74 folded over at its ends and secured to tape backing 72 by a double layer of pressure-sensitive adhesive 76 to form Y-bond 66 and end tab 62.

In a preferred adhesive tape 55 such as shown in Figure 4, the manufacturer's end 56 has a longitudinal width of 2.25 inches (5.7 cm) and a lateral length of 0.75 inches (1.9 cm) and the user's end 58 has a longitudinal width of 2.25 inches (5.7 cm) at the longitudinal sides of the diaper, a longitudinal width of 0.75 inches (1.9 cm) at a distance 0.375 inches (0.95 cm) from its distal end 60, and a lateral length of 1.8 inches (4.6 cm).

Figures 6 and 7 are enlarged plan views showing repeating patterns for making fastening tapes 54 having a sinusoidal-shaped user's end 58, but with a flatter profile than those shown in Figure 4. In Figure 6, adhesive tape 55 comprises manufacturer's end 56, user's end 58, distal end 60, and Y-bond 66. In Figure 7, mechanical fastening tape 57 comprises manufacturer's end 56, user's end 58, distal end 60, and hook region 64, which is a separate patch covering substantially all of the underside of user's end 58.

## Claims

1. A disposable diaper comprising:

   a liquid permeable topsheet (12);

   a liquid impermeable backsheet (16), said backsheet being affixed to said topsheet (12);

   an absorbent means (14) for absorbing liquids, said absorbent means being encased between said topsheet (12) and said backsheet (16);

   longitudinal marginal portions (50) adjacent both longitudinal sides (26, 28) of the disposable diaper;

   elastic members (18) operatively associated with both of said longitudinal marginal portions (50);

   a first waist portion (42) and a second waist portion (44); and

   fastening tapes (54) for maintaining said first waist portion and said second waist portion in an overlapping configuration; each of said fastening tapes (54) having a manufacturer's end (56) and a user's end (58), characterised in that said user's end (58) has a longitudinal width of from 3.8 cm to 10.2 cm at the longitudinal sides (26, 28) of the disposable diaper, and in that said user's end (58) has a longitudinal width at its distal end (60) of less than 60% of its longitudinal width at the longitudinal sides (26, 28) of the disposable diaper.

2. A disposable diaper according to claim 1 wherein the user's end (58) of said fastening tapes (54) has a longitudinal width of from 5.1 cm to 7.6 cm at the sides of the disposable diaper.

3. A disposable diaper according to either one of claims 1 and 2 wherein the user's end (58) of said fastening tapes (54) has a longitudinal width at its distal end of less than 40% of its longitudinal width at the longitudinal sides (26, 28) of the disposable diaper.

4. A disposable diaper according to any one of claims 1-3 wherein the user's end (58) of said fastening tapes (54) has a longitudinal width of from 1.3 cm to 3.8 cm at a distance of 0.95 cm from its distal end.

5. A disposable diaper according to any one of claims 1-4 wherein said fastening tapes (54) have a Taber stiffness of less than 3 g•cm, preferably of from 0.5 to 2 g•cm.

6. A disposable diaper according to any one of claims 1-5 wherein said fastening tapes (54) are adhesive tapes.

7. A disposable diaper according to any one of claims 1-5 wherein said fastening tapes (54) are mechanical fastening tapes.

8. A disposable diaper according to either one of claims 5 and 7 wherein said fastening tapes (54) are refastenable and further comprise a release tape, Y-bond and end tab.

9. A disposable diaper according to claim 6 or claim 8 when dependent thereon wherein the user's end (58) of said fastening tapes (54) has a sinusoidal-like shape.

10. A disposable diaper according to claim 7 or claim 8 when dependent thereon wherein the user's end (58) of said fastening tapes (54) comprises a hook region (64).

11. A disposable diaper according to claim 10 wherein the disposable diaper further comprises a loop region on the outside of the second waist portion (44), said loop region being inboard from the longitudinal side edges of the absorbent means (14) by a distance at least equal to the lateral width of the hook region (64) on the user's end (58) of said fastening tapes (54), and said loop region having a longitudinal width of no more than 1.3 cm larger than the longitudinal length of said hook regions (64).

12. A disposable diaper according to claim 11 wherein the user's end (58) of said fastening tapes (54) has a trapezoidal-like shape.

**Revendications**

1. Une couche à jeter comportant :

une feuille de dessus perméable aux liquides (12) ;

une feuille de fond imperméable aux liquides (16), ladite feuille de fond étant fixée à ladite feuille de dessus (12) ;

un moyen absorbant (14) pour absorber les liquides, ledit moyen absorbant étant encastré entre ladite feuille de dessus (12) et ladite feuille de fond (16) ;

des parties de lisière longitudinales (50) adjacentes aux deux bords longitudinaux (26, 28) de la couche à jeter ;

des éléments élastiques (18) associés aux deux dites parties de lisière longitudinales (50) ;

une première partie de ceinture (42) et une seconde partie de ceinture (44) ; et

des bandes de fixation (54) pour maintenir ladite première partie de ceinture et ladite seconde partie de ceinture en une configuration à recouvrement ; chacune desdites bandes de fixation (54) ayant une extrémité pour le fabricant (56) et une extrémité pour l'utilisateur (58), caractérisée en ce que ladite extrémité pour l'utilisateur (58) a une largeur longitudinale allant de 3,8 cm à 10,2 cm sur les bords longitudinaux (26, 28) de la couche à jeter, et en ce que ladite extrémité pour l'utilisateur (58) a une largeur longitudinale à son extrémité distale (60) inférieure à 60% de sa largeur longitudinale sur les bords longitudinaux (26, 28) de la couche à jeter.

2. Une couche à jeter selon la revendication 1, dans laquelle l'extrémité pour l'utilisateur (58) desdites bandes de fixation (54) a une largeur longitudinale allant de 5,1 cm à 7,6 cm sur les bords de la couche à jeter.

3. Une couche à jeter selon l'une quelconque des revendications 1 et 2, dans laquelle l'extrémité pour l'utilisateur (58) desdites bandes de fixation (54) a une largeur longitudinale à son extrémité distale inférieure à 40% de sa largeur longitudinale sur les bords longitudinaux (26, 28) de la couche à jeter.

4. Une couche à jeter selon l'une quelconque des revendications 1-3, dans laquelle l'extrémité pour l'utilisateur (58) desdites bandes de fixation (54) a une largeur longitudinale allant de 1,3 cm à 3,8 cm à une distance de 0,95 cm de son extrémité distale.

5. Une couche à jeter selon l'une quelconque des revendications 1-4, dans laquelle lesdites bandes de fixation (54) ont une raideur Taber inférieure à 3g. cm, de préférence allant de 0,5 à 2g. cm.

6. Une couche à jeter selon l'une quelconque des revendications 1-5, dans laquelle lesdites bandes de fixation (54) sont des bandes adhésives.

7. Une couche à jeter selon l'une quelconque des revendications 1-5, dans laquelle lesdites bandes de fixation (54) sont des bandes de fixation mécanique.

8. Une couche à jeter selon l'une quelconque des revendications 6 et 7, dans laquelle lesdites bandes de fixation (54) sont susceptibles d'être attachées à nouveau et comportent en outre une bande amovible, une liaison en Y et une patte d'extrémité.

9. Une couche à jeter selon la revendication 6 ou la revendication 8, lorsqu'elle est dépendante de celle-ci, dans laquelle l'extrémité pour l'utilisateur (58) desdites bandes de fixation (54) a une forme analogue à une sinusoïde.

10. Une couche à jeter selon la revendication 7 ou la revendication 8, lorsqu'elle est dépendante de celle-ci, dans laquelle l'extrémité pour l'utilisateur (58) desdites bandes de fixation (54) comporte une partie d'accrochage (64).

11. Une couche à jeter selon la revendication 10, dans laquelle la couche à jeter comporte en outre une partie en boucle sur l'extérieur de la seconde partie de ceinture (44), ladite partie en boucle s'étendant vers l'intérieur à partir des bords latéraux longitudinaux du moyen absorbant (14) d'une distance au moins égale à la largeur latérale de la partie d'accrochage (64) sur l'extrémité pour l'utilisateur (58) desdites bandes de fixation (54), et ladite partie en boucle ayant une largeur longitudinale qui ne dépasse pas de plus de 1,3 cm la longueur longitudinale de ladite partie d'accrochage (64).

12. Une couche à jeter selon la revendication 11, dans laquelle l'extrémité pour l'utilisateur (58) desdites bandes de fixation (54) a une forme analogue à une forme trapézoïdale.

## Patentansprüche

1. Eine Wegwerfwindel, welche umfaßt:

   ein flüssigkeitsdurchlässiges Deckblatt (12),

   ein flüssigkeitsundurchlässiges Rückenblatt (16), wobei das genannte Rückenblatt am genannten Deckblatt (12) befestigt ist;

   ein absorbierendes Mittel (14) zum Absorbieren von Flüssigkeiten, wobei das genannte absorbierende Mittel zwischen dem genannten Deckblatt (12) und dem genannten Rückenblatt (16) eingeschlossen ist;

   Längsrandabschnitte (50), welche an beide Längsseiten (26,28) der Wegwerfwindel anliegen;

   elastische Bauteile (18) , welche mit beiden der genannten Längsrandabschnitte (50) wirksam verbunden sind;

   einen ersten Taillenabschnitt (42) und einen zweiten Taillenabschnitt (44); und

   Befestigungsbänder (54) zum Halten des genannten ersten Taillenabschnitts und des genannten zweiten Taillenabschnitts in einer überlappenden Konfiguration; wobei jedes der genannten Befestigungsbänder (54) ein Herstellerende (56) und ein Verwenderende (58) hat, dadurch gekennzeichnet, daß das genannte Verwenderende (58) an den Längsseiten (26, 28) der Wegwerfwindel eine Längsausdehnung von 3,8 cm bis 10,2 cm hat, und dadurch, daß das genannte Verwenderende (58) an seinem distalen Ende (60) eine Längsausdehnung von weniger als 60 % seiner Längsausdehnung an den Längsseiten (26, 28) der Wegwerfwindel hat.

2. Eine Wegwerfwindel nach Anspruch 1, bei welcher das Verwenderende (58) der genannten Befestigungsbänder (54) eine Längsausdehnung von 5,1 cm bis 7,6 cm an den Seiten der Wegwerfwindel aufweist.

3. Eine Wegwerfwindel nach einem der Ansprüche 1 und 2, bei welcher das Verwenderende (58)der genannten Befestigungsbänder (54) an seinem distalen Ende eine Längsausdehnung von weniger als 40 % seiner Längsausdehnung an den Längsseiten (26, 28) der Wegwerfwindel hat.

4. Eine Wegwerfwindel nach einem der Ansprüche 1 bis 3, bei welcher das Verwenderende (58) der genannten Befestigungsbänder (54) in einem Abstand von 0,95 cm von seinem distalen Ende eine Längsausdehnung von 1,3 cm bis 3,8 cm hat.

5. Eine Wegwerfwindel nach einem der Ansprüche 1 bis 4, bei welcher die genannten Befestigungsbänder (54) eine Taber-Steifigkeit von weniger als 3,9 cm, vorzugsweise von 0,5 bis 2,9 cm, hat.

6. Eine Wegwerfwindel nach einem der Ansprüche 1 bis 5, bei welcher die genannten Befestigungsbänder (54) Klebebänder sind.

7. Eine Wegwerfwindel nach einem der Ansprüche 1 bis 5, bei welcher die genannten Befestigungsbänder (54) mechanische Befestigungsbänder sind.

8. Eine Wegwerfwindel nach einem der Ansprüche 6 und 7, bei welcher die genannten Befestigungsbänder (54) wiederbefestigbar sind und weiters ein Freigabeband, eine Y-Klebung und eine Endlasche umfassen.

9. Eine Wegwerfwindel nach Anspruch 6 oder Anspruch 8, wenn davon abhängig, bei welcher das Verwenderende (58) der genannten Befestigungsbänder (54) eine Sinusbogen-ähnliche Form hat.

10. Eine Wegwerfwindel nach Anspruch 7 oder Anspruch 8, wenn davon abhängig, bei welcher das Verwenderende (58) der genannten Befestigungsbänder (54) einen Hakenbereich (64) umfaßt.

11. Eine Wegwerfwindel nach Anspruch 10, bei welcher die Wegwerfwindel weiters an der Außenseite des zweiten Taillenabschnitts (44) einen Schlaufenbereich umfaßt, wobei sich der genannte Schlaufenbereich in einer der Querbreite des Hakenbereiches (64) am Verwenderende (58) der genannten Befestigungsbänder (54) mindestens gleichen Distanz von den Längsseitenrändern des absorbierenden Mittels (14) einwärts befindet und wobei der genannte Schlaufenbereich eine Längsausdehnung von nicht mehr als um 1,3 cm größer als die Längslänge des genannten Hakenbereiches (64) hat.

12. Eine Wegwerfwindel nach Anspruch 11, bei welcher das Verwenderende (58) der genannten Befestigungsbänder (54) eine trapezoide Form hat.

## Fig. 1

## Fig. 2

11

# Fig. 3

# Fig. 4

# Fig. 5

## Fig. 6

## Fig. 7

55

58

66

56

60

60

57

58

64

56